# EUROPEAN PATENT APPLICATION

(11) **EP 4 667 557 A1**
(43) Date of publication of application: **24.12.2025**
(21) Application number: 24183743.4
(22) Date of filing: 21.06.2024
(51) Int. Cl.: C12M 1/32, C12M 1/00

(54) **CASSETTE WITH A CONVEYOR FOR INCUBATION OF CELLS**

(71) Applicant: Danmarks Tekniske Universitet, 2800 Kgs. Lyngby (DK)
(72) Inventor: KROMANN, Emil Boye, 2800 Kgs. Lyngby (DK)
(74) Representative: Zacco Denmark A/S

(57) **Abstract**

The present disclosure relates to a cassette comprising a conveyor, a driving mechanism, and a medium handling mechanism. The cassette comprises an optical window configured to allow at least one imaging light beam to illuminate at least a portion of the conveyor at an illumination region. The medium handling mechanism is configured to provide a medium to the conveyor and the driving mechanism is configured to move the conveyor. The conveyor is configured to move through the illumination region. The cassette and the conveyor are configured as an incubator for cells comprised in the medium.

## Description

### TECHNICAL FIELD

The present disclosure relates to a cassette with a conveyor for incubation of cells. More specifically, the disclosure relates to the cassette as defined in the introductory parts of claim 1.

### BACKGROUND ART

Cell therapy requires large scale production of therapeutic cells. This process can start with one (or a relatively few) cells that are expanded into billions of cells. Before the cells are delivered to a patient, it is valuable to confirm that every single cell in the batch is benign, i.e., that each individual cell has developed/specialized into the desired cell type with sufficient potency to provide efficient treatment. Images from an optical microscope would allow to determine whether cells are benign or not. However, when billions of cells are to be screened, the main challenges are the insufficient speed of available equipment and the lack of optical access to cells during expansion. Particularly for adherent cells (i.e., cells that naturally anchor onto rigid substrates), suitable high-throughput screening systems are missing. Existing systems for moving cells between their routine-growth environment and an imaging/screening system are too slow. Additionally, these systems may damage and perturb the cells through sheer stress (resulting from rapid fluid flow around the cells), or through exposure to other unfavourable conditions (e.g., unsound oxygen balance) or chemicals (e.g., trypsin). Therefore, there is a need for a system which will enable fast, and minimally perturbing, screening of the cells while also allowing cell production.

### SUMMARY OF THE INVENTION

It is an object of the present disclosure to mitigate, alleviate or eliminate the above-identified deficiency and disadvantage in the prior art and solve at least the above-mentioned problem.

In particular, it is an object of the embodiments of the present invention to provide a cassette enabling cell production and simultaneous cell screening, thus opening doorways to new research avenues in cell therapy and expansion of therapeutic cells.

It is a further object of the embodiments of the present invention to provide a cassette for high-throughput screening of cells and their characterisation.

It is a yet further object of the present invention to provide a cassette which would enable removal of unwanted cells.

It is a yet further object of the present invention to provide a cassette which would enable interfacing with a series of external systems and/or auxiliary instruments.

According to a first aspect there is provided a cassette comprising a conveyor, a driving mechanism, and a medium handling mechanism. The cassette comprises an optical window configured to allow at least one imaging light beam to illuminate at least a portion of the conveyor at an illumination region. The medium handling mechanism is configured to provide a medium to the conveyor. The conveyor is configured to move through the illumination region. The driving mechanism is configured to move the conveyor. The cassette and the conveyor are configured as an incubator for cells comprised in the medium.

Having a cassette in accordance with the first aspect of the present invention will enable large-volume production of biological cells and simultaneous image-based screening of such large volumes of bio-cells. The screening of every single cell produced in the cassette is performed without removing the cells from the aseptic environment within the cassette. The screening of large volumes of cells can be performed at the same pace as cell growth and any degenerative (unwanted) cell can be identified during the screening and it can be ensured that only good cells are considered and harvested. The screening of cells can also be used to document that the cell population is sufficiently potent and pure for therapeutic use. In the following text, the terms biological cells, bio-cells, or only cells will be used interchangeably, all referring to basic building blocks of living organisms and/or biological entities of similar nature, e.g., bacterial cells or algal cells.

In the present context, the cassette may be understood as an enclosed container, case, chamber, or a cartridge that has controlled atmosphere/environmental conditions, e.g. controlled pressure, controlled temperature, etc. The cassette may be an incubator. The cassette should be large enough to accommodate at least a conveyor and a mechanism for rolling the conveyor there within. However, the size and volume of the cassette may vary and may be changed depending on the application and a demand on a number of cells to be produced, inspected, and incubated.

The conveyor of the present invention is to be understood as a substrate for handling the medium and the cells contained in the medium. The conveyor may be a mechanical apparatus suitable for cell growth. The conveyor may be configured to allow replacement of the liquid part of the medium around the cells. The replacement of the liquid part of the medium may occur without introducing sheer stress to the cells contained in the medium. The conveyor is arranged in the cassette such that it can move the cells within the cassette.

The conveyor may be made from an optically transparent material. The conveyor may be transparent in the visible regime 400-700 nm. Other wavelength ranges may also be chosen depending on the light sources and signals being used for imaging. The choice of transmissible wavelength ranges would accommodate the wavelengths of the light sources used for imaging, cell elimination, and reading of position encoders on the conveyor.

The conveyor may be configured to bend, such as bend around the driving mechanism, i.e. around elements of the driving mechanism. In other words, the conveyor is made of a flexible and bendable material allowing it to bend around the driving mechanism. The conveyor may be configured to hold the medium and/or cells thereon, such as by adhesion. The conveyor may be configured to allow storage and access of the cells adhered thereto. The conveyor may comprise wells, and the medium with the cells will typically fill up the wells while the cells will attach to bottom of the wells. The conveyor may be thin and flexible, allowing to accommodate a large surface area of the conveyor within a relatively small volume of the cassette, e.g., by rolling the conveyor onto itself, like a roll of tape or thread. The conveyor may be partially immersed in gas and partially immersed in liquid. For example, the part of the conveyor that is rolled onto a reel may be immersed in liquid, while parts of the conveyor suspended between reels may be exposed to a gas. The part of the conveyor that is rolled onto a reel may also be only partially immersed in liquid and partially in gas (e.g., because the reel is partially immersed in liquid and partially in gas).

The medium typically comprises cells dispersed therein. In particular, the medium that is initially applied onto the conveyor may be a cell suspension or a suspension culture, i.e., it comprises cells, while the medium that is applied to the container after the cell growth is initiated may not comprise cells. The medium may comprise a fluid, such as liquid. It may be in the form of a liquid suspension.

The driving mechanism is to be understood as a mechanism responsible for moving the conveyor within the cassette. The driving mechanism may comprise one or more pulleys, one or more reels, one or more rotational wheels, one or more conveyor belt rolls (e.g., idler rolls, drums), and/or one or more motors to drive those. Some such pulleys/reels/wheels/rolls may be driven by a motor while some may serve as passive guides/supports for the conveyor. For example, the driving mechanism may use a motor to power a pulley, which in turn causes the pulley to move the length of the conveyor. The force that drives the conveyor may be produced by friction between the pulley and the underside of the conveyor's moving surface. The force that drives the conveyor may also be produced by friction between the pulley and multiple sides of the conveyor's moving surface. This frictional force therefore leads to movement of the conveyor along a given route. In cases where the conveyor comprises at least two layers, pulleys may be configured (positioned) to peel the layers apart and/or realign them on top of each other, e.g. by running one layer of the conveyor over one pulley (or set of pulleys) and another layer of the conveyor over another pulley (or set of pulleys). Pulleys may interface with the conveyor via sprockets on the pulleys and sprocket holes on the conveyor, thus guiding the motion of the conveyor. Alternatively, or additionally, one end of the conveyor may be affixed to a pulley (or reel), thus enabling motion of the conveyor by rotating the pulley (or reel) and rolling the conveyor onto the reel. Similarly, both ends of the conveyor may be affixed to one or more reels, allowing the conveyor to move in both directions. The driving mechanism may define the speed and direction of motion of the conveyor through the illumination region.

The medium handling mechanism may be considered as a loading device that ensures loading of the medium onto the conveyor, as well as loading of the medium off the conveyor. The medium handling mechanism providing the medium to the conveyor may comprise a compartment configured as a reservoir for the medium. The medium handling mechanism may ensure that the cells in the medium are initially delivered to the conveyor. When the cells are initially delivered to the conveyor, the cells will attach to the conveyor, so-called cell-seeding. Once the cells have attached to the conveyor, the medium, or more precisely a liquid portion of the medium, around the cells may occasionally be replaced while the cell population expands on the conveyor, so-called cell-expansion. Cell-seeding and replacement of the medium during cell expansion may occur while the conveyor travels between, e.g., two reels. Medium replacement may also occur while the conveyor is rolled up onto the reels. The medium handling mechanism may comprise a reservoir for cell harvesting after screening of cells.

The cassette may comprise a control mechanism. The control mechanism may be configured to control environmental parameters in the medium and in the cassette. The control mechanism may comprise one or more devices configured to control the environment inside the cassette. The control mechanism may also comprise one or more sensors configured to measure parameters of the environment inside the cassette. Alternatively, the control mechanism may be connected to the cassette through an interface point arranged on the cassette.

The cassette comprises an optical window configured to allow at least one imaging light beam to illuminate at least a portion of the conveyor at an illumination region.

The conveyor may be made from a material that is transparent to the imaging light beam, allowing the light beam to reach the cells on/inside the conveyor and allowing any signal elicited by the light beam to escape the conveyor.

In the present context, the term "optical window" is to be understood as a portion of the cassette that is transparent to the imaging light beam. This portion of the cassette may be in a form of a glass window, or an opening, or made of a material that is transparent for the wavelengths of the imaging light beam. In some cases, the entire cassette may be made of a material that is transparent for the imaging light beam to thereby ensure flexibility of arranging the cassette with respect to the imaging beam.

The imaging light beam will typically be arranged outside of the cassette. The imaging light beam may comprise a plurality of light beams, interference patterns, or the like. The imaging beam enables observation of the medium and the cells comprised in the medium while the medium is moved along the conveyor and/or together with the conveyor. The imaging light beam may be selected based on the cells to be screened. Various imaging techniques and therefore imaging light beams can be used to image the cells in the cassette, such as through fluorescence techniques or Raman spectroscopy. The imaging light beam can be used to monitor the cells and analyze them both quantitatively and qualitatively, e.g., to simply count the cells in the cassette and/or to determine their quality, e.g., their morphology, which may, in turn serve as an indicator of therapeutic potency of cells intended for use in cell therapy. As such, this monitoring of cells may serve as a potency assay for therapeutic cells. Depending on the technique selected for cell imaging, the speed of the conveyor may be different to allow for screening of all the cells present on the conveyor.

The illumination region is the region where the imaging light beam entering the cassette through the optical window crosses with the conveyor. The illumination region may move along/across the conveyor if the imaging light beam is moved, if the conveyor is moved, or if the whole cassette (containing the conveyor) is moved relative to the imaging light beam.

The driving mechanism is configured to move the conveyor through the illumination region. In other words, any element forming part of the driving mechanism is arranged such that it ensures that the conveyor passes through the area that can be illuminated by the imaging light beam.

The driving mechanism is configured to move the conveyor to thereby move the medium arranged on the conveyor. In other words, the conveyor is configured to move the medium (and the cells comprised in the medium) through the cassette and through the illumination region. As the conveyor moves through the cassette and also with respect to the illumination region, the medium also moves through the cassette and also with respect to the illumination region. As the medium moves together with the conveyor, the bio-cells to be interrogated also move with respect to the illumination region thereby ensuring that all the bio-cells on the conveyor are screened. The bio-cells typically adhere to the conveyor, thus ensuring the same movement of the bio-cells through the cassette as the movement of the conveyor. Medium (including fluid portions of the medium and suspended bio-cells) can be trapped in wells or pockets on/in the conveyor, thus ensuring the same movement of medium through the cassette as the movement of the conveyor.

The control mechanism may be configured to control environmental parameters in the medium and in the cassette. The control mechanism may also be configured to control environmental parameters in a close proximity of the conveyor as well as at the conveyor. The control mechanism may control environmental parameter of air/gas and liquids comprised in the cassette. One or more devices and sensors of the control mechanism responsible for controlling the atmosphere/environment in and around the cassette may perform measurements of the relevant environmental parameters at scheduled times and perform adjustments of those when necessarily. The one or more devices and sensors of the control mechanism may also be placed outside of the cassette and connected to the cassette via one or more interface points arranged on the cassette.

The environmental parameters may include temperature, humidity, pressure, CO₂ content, oxygen content, etc. of the atmosphere/environment inside the cassette. When it comes to the medium, temperature, CO₂ content, and oxygen content may also be measured.

The cassette and the conveyor are configured as an incubator for cells comprised in the medium, thus allowing cell growth, cell differentiation, and cell expansion on or in the conveyor within the cassette. In other words, the cassette is configured to allow cell growth, differentiation, and/or expansion.

The cassette may be configured for image-based screening of cells contained in the medium. The medium may comprise a fluid and cells dispersed in the fluid. However, once the cells have attached to the conveyor, the new medium introduced from the medium handling mechanism may not necessarily contain cells. In this case, the new medium may contain only nutrients, gas, and reagents needed for maintaining the cell culture and for the further cell expansion. Additionally, the media may contain contrast agents (e.g., fluorescent markers) necessary for imaging.

The conveyor may comprise a plurality of layers. The layers may be made from flexible materials. Having the conveyor made from a flexible material ensures that it can be rolled onto a reel. At least two layers may be configured to capture and guide the medium therebetween. As the layers capture the medium (with liquid and cells contained therein) therebetween, the medium will be equally distributed along the conveyor and only a small portion of medium travels along with the cells as the conveyor moves and passes through the illumination region. Such distribution of the medium on the conveyor eliminates or at least drastically reduces problems relating to sheer stress that surrounding fluid of the medium might exert on the cells.

The conveyor may comprise wells configured to contain a volume of the medium, and thereby cells as the medium comprises the cells. The bottom of the wells may be configured for attaching the cells comprised in the medium. The attachment to the bottom of the wells may be enabled by the conveyor itself, i.e., the conveyor is uncoated, e.g., made from a biocompatible material, making coating unnecessary. In some examples, however the conveyor may comprise a biocompatible coating ensuring cell adhesion. In some cases, the conveyor may be coated with a film which the cells will adhere to. In some further cases, the wells only may be coated with a material suitable for cell adhesion, such as Polystyrene. If the conveyor comprises layers, at least one layer may comprise the wells, and these wells may have adhesive properties with respect to the cells. The wells intended to contain cells may exist in a cell layer of the conveyor. Here, one or more cells are isolated while still immersed in a predefined volume of the fluid part of the medium. The wells are typically shallow, having the depth of up to 1 mm and may take any shape. Typically, the wells are rectangular with a volume of up to 100 mm³, e.g., 10×10×1 mm. Having the conveyor with wells allows to observe every single cell adherent to the bottom of the well without introducing sheer stress or removing cells from their aseptic growth environment.

The conveyor may comprise pores, e.g. perforations, and wells. The wells may be configured as reservoirs for the medium, either containing cells or not containing cells, while the pores may be configured to enable medium exchange within the conveyor. If the conveyor comprises layers, at least one layer may comprise pores and/or wells, the wells acting as reservoirs. In this case, the pores may be configured to enable medium exchange between wells in the layers of the conveyor. The wells are typically shallow, and may at most be 1 mm deep, and the pores may be at most 1 mm in diameter. In some embodiments, at least one layer (the so-called gate layer) may comprise pores and at least one layer (the so-called cell layer) arranged below the gate layer may comprise the wells with cells. In this way, medium exchange is enabled between the cell layer and the gate layer. In one embodiment, the conveyor may have two layers. A first layer may be with shallow wells containing a predetermined volume of medium and a coated (e.g., polystyrene) or uncoated bottom for cell adhesion. This first layer may be referred to as the cell layer. A second layer may be with shallow wells (e.g. 1 cm² surface area, but not necessarily square shaped) and relatively large pores (1 mm in diameter) at the bottom of the wells. The second layer typically does not contain cells but rather allows for a quick replacement of the medium and/or for maintaining gas and nutrient concentrations without exposing the cells in the first layer to sheer stress. This second layer may be referred to as the gate layer. Thereby, the conveyor can always be liquid filled liquid-immersed within an aseptic enclosure. Such a conveyor also allows screening of every single adherent cell (those adherent to the bottom of the wells in the cell layer) without introducing sheer stress on the cells or removing cells from their aseptic growth environment.

In the embodiments in which the conveyor comprises two or more layers, the layers may be configured to move relative to each other. This can allow to adjust the alignment between wells/pores in different layers of the conveyor. As the layers move with respect to each other, they may be switching between the state of allowing medium exchange, e.g. via the pores, and disallowing medium exchange there between. This mechanism of allowing and disallowing medium exchange will protect the cells from sheer stress, caused by, e.g., liquid turbulence, while the conveyor is moving (e.g., while the conveyor is suspended between the reels) and/or while the cell-free medium in reservoir-wells is rapidly replaced. Notably, because the conveyor is typically made from a flexible material, some parts of the conveyor may be in a state disallowing media exchange between layers (e.g., the part that is in motion and suspended/exposed between reels), while other parts of the conveyor are in a state allowing media exchange between layers (e.g., the much larger parts of the conveyor that are rolled onto reels).

In the embodiments in which the conveyor comprises two or more layers, the layers may be configured to be peeled apart and re-aligned on top of each other. This allows interfacing with parts of the conveyor that would otherwise be inaccessible, e.g., wells in layers of the conveyor that would otherwise be covered by another layer above it. Peeling the conveyor apart can be useful when seeding cells onto the conveyor or harvesting cells from the conveyor. Peeling the conveyor apart can also be useful for reducing the number of layers in the conveyor that the imaging system needs to capture images through.

The conveyor may comprise a plurality of walls arranged along the length of the conveyor thereby forming a plurality of pockets along the conveyor. The walls may be integral parts of the conveyor and therefore made of the same material as the rest of the conveyor. The pockets may be configured to hold the medium therein, and thus the cells. The walls prevent flow along the length of the conveyor thereby effectively dividing the conveyor into a series of medium-filled pockets. In the case of a conveyor formed of a single layer, the pockets formed by the walls, may effectively have the same function as the wells mentioned above. This can also be the case if the conveyor is a belt but also if the conveyor is a tube. In the case of the conveyor in a form of a tube, the pockets form separate compartments formed in the tube. The walls may also be provided between two layers such that the pockets are formed by two side walls and two layers, one forming the top and one forming the bottom of the pocket. The cells will typically only adhere to the bottom of the pocket and not to the walls thereby ensuring that every single cell is screened.

The invention disclosed here may exploit that rotation of the reels of the driving mechanism may generate a centripetal force on/within the conveyor. This will effectively push the cells towards one side of the conveyor. If this side is the bottom of wells, then the centripetal force can push cells in suspension onto the bottom of the well before they adhere to the conveyor, thus ensuring that cells adhere only to the bottom of wells. Similarly, if the conveyor is flipped relative to the reel (such that the centripetal force induces motion out of the wells), then constituents of the media that are not adhering to the conveyor (e.g., cells debris or cells that are not adhering) may be pushed away from the bottom of wells. Subsequent replacement of medium may then remove these cells from the cassette.

The cassette may comprise at least one interface point configured for interfacing the cassette and an external system and/or an auxiliary system or device. One of the interface points may be in a form of ports for gas and/or liquid exchange. These ports may also serve for cell seeding and/or for cell harvesting. One of the interface points may be in a form of additional optical windows for further imaging of the medium and/or for radiation-based removal of unwanted cells. One of the interface points may be in a form of heating and/or cooling pads for temperature control. One of the interface points may be in a form or a mechanical interface for interfacing the cassette with a mechanism for driving the conveyor belt, such as a mechanism for rotating one or more pulleys and/or reels. This mechanical interface may use magnets, e.g. in a stepper-motor configuration, to rotate the pulleys inside the cassette, thus allowing to move the conveyor, without opening the cassette, which would cause a risk of contamination. One of the interface points may be in the form of an electric connection for powering and communicating with sensors or actuators inside the cassette. One of the interface points may be in the form of an optical window allowing to read position encoders on the conveyor. One of the interface points may be in the form of a mechanical actuator for moving the cassette relative to the imaging system, thus directing the at least one imaging light beam through different parts of the optical window (and thus onto different parts of the conveyor). Providing the cassette with multiple interface points allows for further functionalities of the cassette and additional sensing/control of the atmosphere/environment inside the cassette.

The conveyor may be configured to bend around the driving mechanism. For instance, the conveyor may be rolled onto one or more reels like an analogue film roll, the reels representing part of the driving mechanism. To allow bending of the conveyor, the conveyor is bendable and flexible, and typically made of bendable/flexible materials, for example polyester or polyethylene terephthalate plastics (also known as PET).

The conveyor may be a flexible/bendable conveyor belt or a conveyor tube. The conveyor belt may include tubes and reservoirs for local liquid/gas exchange. The conveyor belt may have structures with well-defined shapes, allowing to optimize and calibrate the imaging system. The conveyor belt may have distinctive marks that encode position on the belt, allowing to identify and re-image or treat a specific cell or area of the belt. Having the conveyor in the form of a conveyor belt ensures a continuous translation of the medium and its speed of transport may be controlled depending on the speed of imaging, improving overall efficiency of the cell-screening process. Similar features may be implemented if the conveyor is a conveyor tube.

The conveyor may be a hollow fiber with optical access, such that the cells can be screened by an optical beam.

The control mechanism may be configured to control at least one of temperature, pressure, Ph, gas composition, or humidity of gas and/or liquid around the conveyor. The control mechanism may also be configured to control temperature, pressure, Ph, gas composition, or humidity of gas and/or liquid of the atmosphere/environment inside the cassette. In some embodiments, the conveyor may be immersed in a liquid and the control mechanism may measure and control temperature, pressure, Ph, or liquid composition of the liquid. In some other embodiments, the conveyor may be immersed in a gas, and the control mechanism may measure and control temperature, pressure, Ph, composition of the gas, or humidity of the gas. The control mechanism may comprise a list of preferred parameter values and therefore ensure that all the parameters around the conveyor and in the cassette are maintained as close to the preferred values as possible. In that way, optimal conditions for the cell expansion are maintained.

The conveyor may be configured to contain a monolayer of cells comprised in the medium. Alternatively, or additionally, the conveyor may be configured to contain clusters/aggregates of cells comprised in the medium. The conveyor may comprise a layer of scaffolding material that may be permeated by the medium. In some cases, the formation of cell clusters may be promoted by introducing a hydrogel (or other scaffolding material that may be permeated by media) along with the cells, when they are seeded onto the conveyer. Alternatively, or additionally, the conveyor may be configured to contain cells suspended in the media, or single cells suspended in a hydrogel (or other scaffolding material that may be permeated by media). For example, a monolayer of cells may adhere and expand on the bottom of wells in the conveyor as described previously. Similarly, clusters of cells (e.g., organoids or spheroids) may attach to the bottom of wells. Such cells or clusters of cells may also remain affixed near the bottom of wells via immersion into a scaffolding material (e.g., a hydrogel). Non-adherent individual cells and/or clusters of cells may also be trapped inside wells by placing these entities (cells and/or cell clusters) in the previously described wells in the cell-layer of a two-layer conveyor and making the pores in the gate layer so small that the suspended entities cannot escape via the pores. This would contain the entities inside the wells of the cell-layer, even if they are suspended in medium. As the pores would need to be small (<20 µm) to prevent individual cells from escaping, this strategy may be best suited for containing/trapping larger clusters of cells, such as organoids and/or spheroids. Regardless of whether the cells are arranged as a monolayer or in clusters, and regardless of whether these entities are in liquid suspension or trapped in a scaffolding material or adherent to the conveyor, the imaging beam and the driving mechanism and their speeds will ensure that each and every cell may be accessible to the imaging system, thus allowing screening, counting, and analysis of the cells.

The conveyor may be configured to allow cells contained in the medium to attach to the conveyor. In some implementations the conveyor may comprise a coating suited for cell adhesion. Adhesion reduces cell loss. Additionally, adhesion helps in a better distribution of the cells along the conveyor. As the cells adhere to the conveyor they can assume the morphologically distinct shape and behavior of adherent cells of their given cell type (or cell level of differentiation/specialization). As such, images capturing the shape (or other structural characteristics or spatial distributions of contrasts) of/around these adherent cells may be used to characterize their cell type and/or level of differentiation/specialization (including their potency as therapeutic cells). Images of the same cells in suspension may not provide the same information, as cells in suspension tend to assume a spherical shape, regardless of their cell type and level of differentiation (making it more difficult to link cell morphology to characteristics like cell type and/or therapeutic potency).

The conveyor may comprise machine-readable position-encoding markers that allow to associate captured image information to specific locations on the conveyor. These position-encoding markers may be bar-code printed along the edge of the conveyor, alternatively (or additionally) they may be fiducial markers (e.g. point-like structures, constellations, or bar-codes) close to the region of the conveyor where cells attach. Such position encoding markers may be imaged directly by the imaging system that is also geared for imaging bio-cells. Such position markers may also be deciphered by a separate (typically optical) mechanism. Recognition of the position-encoding markers will facilitate time-lapse imaging of the conveyor (e.g., repeated or interleaved imaging of the same well-defined regions of the conveyor). This will also facilitate revisiting of specific regions of the conveyor for other reasons than imaging. For example, screening of the conveyor may be followed by data/image analysis, leading to a decision to revisit a specific part of the conveyor where imaging failed (and then re-imaging this area) or where intervention is needed, e.g., removal/elimination of unwanted cells. Notably, the position-encoding markers allow to identify (and, thus, re-visit) specific regions of the conveyor. In this regard, it is advantageous if the bio-cells are adherent (or confined to relatively small wells/pockets), such that the bio-cells will not move (far) between the time-point where they are imaged/screened and the time-point where they are re-visited. The position-encoding markers may also be used by separate systems geared to interface with the conveyor either before it is assembled into the cassette or after it might be removed from the cassette and treated as a separate entity. For example, a separate system might use screening-data (obtained with the invention disclosed here) in conjunction with the position-encoding markers to automatically remove those parts of the conveyor that contains unwanted cells, i.e., removal of unwanted cells may also take place in a separate system, once expansion is complete. This may, however, require maintenance of aseptic conditions also outside the cassette.

The conveyor may comprise features (markers) configured for calibrating the associated imaging system. These features may be point-like structures or other structures with predefined shapes. For example, fluorescent beads, Silicate beads, polystyrene beads (or other beads eliciting a pronounced Raman signal) or gold nano-particles (effective light scatters) may be embedded into or seeded onto the conveyor. These features may also be air-bubbles inside the conveyor or well-defined divots pressed into the conveyor. When these features are imaged by the associated imaging system, the imaging system may adapt such that the predefined features are reproduced (imaged) faithfully/accurately. Such a feedback mechanism will ensure that bio-cells (with unknown shapes) are also reproduced (imaged) faithfully/accurately. Hence, the presence of the predefined features on/within the conveyor can be useful for optimizing and validating imaging of cells on/within the conveyor.

The conveyor may comprise embedded, i.e. built-in, point-like features (sources) and/or features with predefined shapes that can be imaged with the imaging system, such as fluorescent beads and/or gold nanoparticles, which are efficient light scatterers. Those may be embedded into the conveyor. Such point-sources are beneficial for compensating optical aberrations in images. This compensation may happen computationally in post-processing and/or actively during imaging. Imaging may start by imaging a feature known to be point-shaped (a sub-diffraction sized feature) and then checking that the captured feature appears like a point (or point-spread function) in the resulting image. Finally, the imaging system may be tuned to make the known point-shaped feature look as much like a point as possible. This is typically optimized in a closed feedback loop. This can be useful if, for example, the conveyor is not perfectly uniform along its length. For example, one stretch/region of the conveyor may be slightly thicker than another due to non-ideal manufacturing tolerances. In this case, the image quality may change slightly between different regions of the conveyor (as different conveyor thicknesses can introduce different optical aberrations). When the markers are distributed throughout the conveyor, images may be optimized for such separate regions of the conveyor. Effectively, the image of a given cell may be optimized and/or validated based on the markers (or features with known shapes) that are closest to said cell.

The at least one interface point may be configured for selective removal/elimination of unwanted cells. The unwanted cells may be identified as having certain deviating characteristics from characteristics of healthy cells. The interface point configured for removal (or elimination) of unwanted cells may be in a form of an optical access or a mechanical access, i.e., the unwanted cells can be removed from the conveyor either mechanically or optically. In other words, at least one interface point may be configured for an optical access and/or for a mechanical access to thereby enable selective removal of unwanted cells. In some cases, the at least one interface point may be configured for a magnetic access. In the case of the optical cell removal, the cassette may comprise a second optical window, and an optical beam can be arranged outside the cassette entering the cassette through the second optical widow to illuminate the medium and/or conveyor at a second illumination region. The optical beam may have a predefined wavelength which ensures that the unwanted cells are removed (or destroyed/killed) from the conveyor. For example, the at least one interface point may allow an optical tweezer to remove unwanted cells. Alternatively, the at least one interface point may allow to project a high-powered laser beam onto unwanted cells, thereby destroying the unwanted cells (possibly including parts of the conveyor that the unwanted cells might adhere to). Non-adherent cells, cell debris, and other debris may be removed by replacing the medium in the conveyor. An alternative way to remove/neutralize unwanted cells would be to embed the unwanted cells in a substance that isolates the cells, preventing such cells from expanding further and preventing them from being harvested. For example, a mechanical interface point may be used to deposit a resin into those wells that contain unwanted cells. By having an interface point on the cassette that allows removal/isolation/neutralization/elimination of the unwanted cells, it is ensured that only healthy cells are existing on the conveyor and in the cassette, or (more importantly) only healthy cells are harvested and used for treatment.

At least one interface point may be configured for a magnetic access. Such an interface point may form part of the driving mechanism as a magnetic source may be directed through the magnetic access to thereby drive the conveyor. In particular, the magnetic source may drive the reels and/or pulleys in a similar manner as a rotary stepper motor.

At least one interface point may be configured for cell seeding and cell harvesting. In some implementations, temporary removal/opening (under sterile or aseptic external conditions) of a hatch in the cassette may provide this interface point.

The cassette may be configured to provide a sterile or aseptic enclosure for the medium. Naturally, as the cassette provides a sterile or aseptic enclosure for the medium, the cells contained in the medium are also provided with the sterile or aseptic enclosure. The sterile or aseptic enclosure may be necessary for adequate incubation of the cells and their growth. The sterile or aseptic enclosure prevents contamination and ensures successful growth and development of the bio-cells.

The conveyor may be made from a biodegradable material. Having a biodegradable conveyor allows for delivering cells into patients along with parts of the conveyor. Alternatively, a thin and detachable or biodegradable film (or coating) arranged on the surface of the conveyor which the cells may adhere to may serve the same function. Alternatively, a thin and dissolvable film (or coating) may allow to release the cells from the conveyor by dissolving those parts of the conveyor that the cells adhere to. This allows to selectively release those cells that are attached to the imaging-friendly regions at the bottom of wells, i.e., those regions that are selectively coated with a dissolvable film (or coating). Thus, cells which accidentally adhere elsewhere on the conveyor, i.e., cells that might not have been screened, would not be released (and harvested) upon dissolving of the film. Likewise, those cells deemed suitable for harvesting may be selectively detached and harvested, while leaving the unwanted cells attached to the conveyor. Selective detachment may, for example, be stimulated by a light beam projected into the cassette, such that the light beam interacts with the conveyor or conveyor-coating to locally release/detach specific cells (rather than eliminating specific cells as described previously). All, these solutions allow to move cells from the conveyor to a patient (or into storage) without exposing the cells to trypsin or similar regents normally used to release adherent cells from the substrate they adhere to.

The cassette and conveyor within may be designed for single use. Alternatively, the cassette may be designed to be reusable (e.g., compatible with sterilization procedures like autoclavation, UV-sterilization, or chemical sterilization). The conveyor may also be replaced between repeated uses of the cassette.

According to a second aspect there is provided a system for production and imaging of bio-cells, the system comprising the cassette of the first aspect and a first light beam source configured to illuminate at least a portion of the conveyor at a first illumination region. The first light beam source is configured to image bio-cells comprised in the cassette. The system further comprises a detection means configured to detect light emitted from the illumination region. The detection means may be configured to generate an image of each cell, the image may be a two-dimensional (2D) image, as well as three-dimensional (3D) image. The system is provided to ensure production as well as a qualitative and quantitative analysis of biological cells. The system may further comprise a second light beam source configured to illuminate at least a portion of the conveyor at a second illumination region, wherein the second light beam source generates a second light beam configured for removal of unwanted cells from the cassette. The unwanted cells may be removed by a high-power laser. The system may further comprise further optical means and/or mechanical means and/or magnetic means configured for observing and analyzing the medium in the cassette. All advantages and features presented in connection to the first aspect of the present disclosure are equally applicable to the second aspect.

According to a third aspect there is provided a substrate for cell growth, the substrate being configured to accommodate a monolayer and/or clusters of cells comprised in a medium. All advantages and features presented in connection to the first aspect of the present disclosure, and in particular in connection to the conveyor, are equally applicable to the third aspect. The substrate may be a conveyor for the cassette of the first aspect. The substrate may be configured to allow cells contained in the medium to attach thereto. The substrate may be made from a biodegradable material. The substrate may be configured to bend. The substrate may be made from a flexible material. The substrate may be made from an optically transparent material. The substrate may comprise a plurality of layers, wherein at least two layers are configured to capture and guide the medium therebetween. The substrate may comprise wells configured to contain a volume of the medium, wherein the bottom of the wells is configured for attaching the cells comprised in the medium, and wherein the wells may be coated with a material suitable for cell adhesion. The substrate may comprise wells and pores, the wells being configured as reservoirs for the medium, the pores being configured to enable a medium exchange within the substrate.

The layers may be configured to move relative to each other, thereby switching between allowing and disallowing medium exchange between layers of the substrate. The substrate may comprise a plurality of walls arranged along the length of the substrate thereby forming a plurality of pockets along the substrate, the pockets being configured to hold the medium.

The present disclosure will become apparent from the detailed description given below. The detailed description and specific examples disclose preferred embodiments of the disclosure by way of illustration only. Those skilled in the art understand from guidance in the detailed description that changes and modifications may be made within the scope of the disclosure.

Hence, it is to be understood that the herein disclosed disclosure is not limited to the particular component parts of the system described since such system may vary. It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context explicitly dictates otherwise. Thus, for example, reference to "a unit" or "the unit" may include several devices, and the like. Furthermore, the words "comprising", "including", "containing" and similar wordings does not exclude other elements or steps. Finally, it is to be understood that every mentioned feature may, or may not be present in one specific embodiment of the present invention. One or more above mentioned features can be combined with the main aspect as described in claim 1.

### BRIEF DESCRIPTION OF THE DRAWINGS

The above objects, as well as additional objects, features and advantages of the present disclosure, will be more fully appreciated by reference to the following illustrative and non-limiting detailed description of example embodiments of the present disclosure, when taken in conjunction with the accompanying drawings.
- Fig. 1: schematically illustrates a cassette according to one embodiment of the disclosure;
- Fig. 2: schematically illustrates a cassette according various embodiments of the disclosure;
- Fig. 3: schematically illustrates a conveyor according to one embodiment of the disclosure;
- Fig. 4: schematically illustrates a conveyor according to yet one embodiment of the disclosure;
- Fig. 5: schematically illustrates a cassette according to different embodiments of the disclosure; and
- Fig. 6: schematically illustrates a system comprising a cassette.

### DETAILED DESCRIPTION

Various embodiments are described hereinafter with reference to the figures. Like reference numerals refer to like elements throughout. Like elements will, thus, not be described in detail with respect to the description of each figure. It should also be noted that the figures are only intended to facilitate the description of the embodiments. They are not intended as an exhaustive description of the claimed invention or as a limitation on the scope of the claimed invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

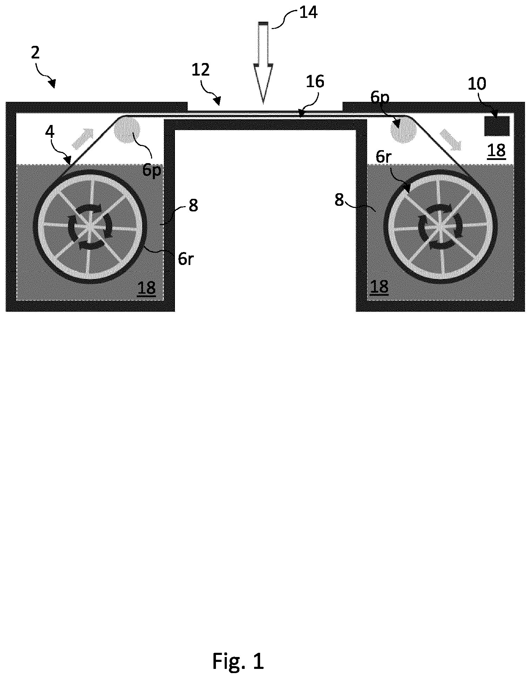

The present disclosure pertains to provide a cassette 2 for growth and screening of bio-cells. Fig. 1 schematically illustrates a cassette according to one embodiment of the disclosure comprising a conveyor 4, a driving mechanism 6, a medium handling mechanism 8, and optionally a control mechanism 10. The control mechanism may alternatively be arranged outside of the cassette and connected to the cassette via an interface point. The cassette 2 comprises an optical window 12 configured to allow at least one imaging light beam 14 to illuminate at least a portion of the conveyor 4 at an illumination region 16. The medium handling mechanism 8 is configured to provide a medium 18 to the conveyor 4. The driving mechanism 6 configured to move the conveyor and the conveyor 4 is arranged such that it moves through the illumination region 16. The control mechanism 10 may be configured to control environmental parameters in the medium 18 and in the cassette 2. The control mechanism 10 may be configured to control at least one of temperature, pressure, Ph, gas composition, or humidity of gas and/or liquid around the conveyor 4. The cassette 2 and the conveyor 4 are configured as an incubator for cells comprised in the medium 18.

The cassette 2 is configured to provide a sterile or aseptic enclosure for the medium 18. The cassette 2 enables large-volume production of bio-cells and simultaneous image-based screening of such large volume bio-cells. Unlike other cell expansion systems, the screening of every single cell produced in the cassette can be performed without removing the cells from the sterile environment of the cassette 2. The screening of large-volume cells can be performed at the same pace as cell growth and any degenerative cell can be marked during the screening and it can be ensured that only good cells are considered and harvested. The cassette layout allows to move cells through a well-defined straight path, such that the imaging system can perform accurate screening of the cells.

Fig. 1 discloses one way of implementing the cassette 2, and in particular one way of implementing the driving mechanism 6, medium handling mechanism 8, and arrangement of the optical window 12. The cassette 2 may comprise a mechanical access to its interior and the conveyor. The conveyor 4 may be made of a flexible material and may thereby be configured to bend around the driving mechanism 6. The conveyor 4 may be made from a biocompatible material which may also be a biodegradable material. The conveyor 4 may be a conveyor belt or a conveyor tube. The conveyor may be configured to contain a monolayer and/or clusters of cells comprised in the medium.

In one implementation, the driving mechanism 6 may be formed by one or more reels 6r and one or more pulleys 6p. The conveyor 4 may be arranged around the reels and further supported by pulleys. The reels and the pulleys rotate and thereby ensure motion of the conveyor 4.

In the example of the cassette illustrated in Fig. 1, the cassette is filled with a liquid part of the medium which covers the reels 6p and the rest of the cassette volume is filled with a gas part of the medium so that the pulleys 6p are surrounded with the gas. In some other cases, the gas part may be covering the reels 6r while the liquid part may be above the gas part thereby covering the pulleys.

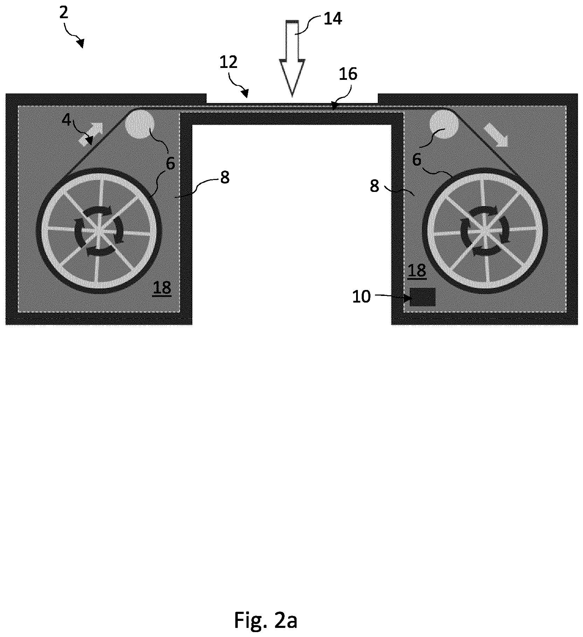

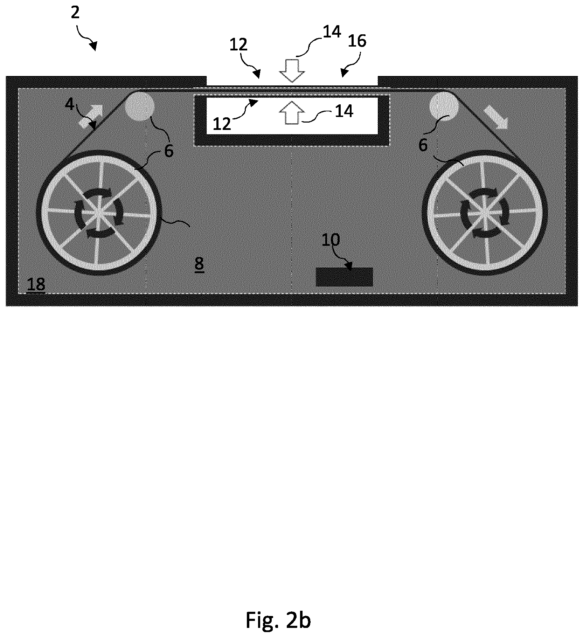

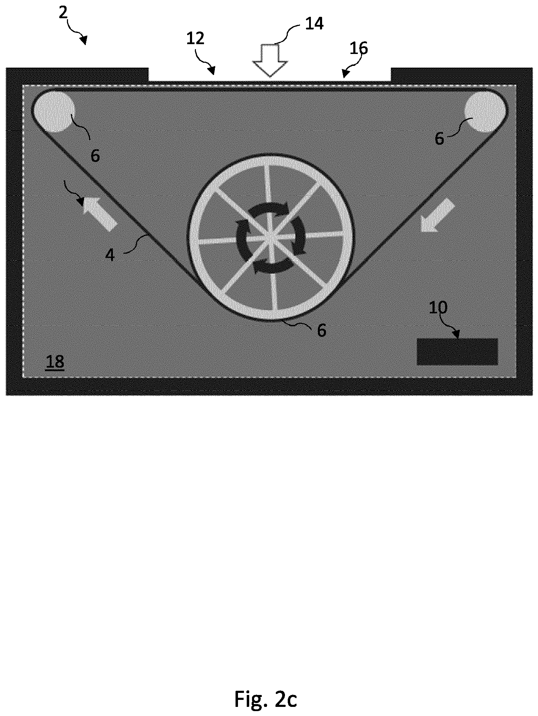

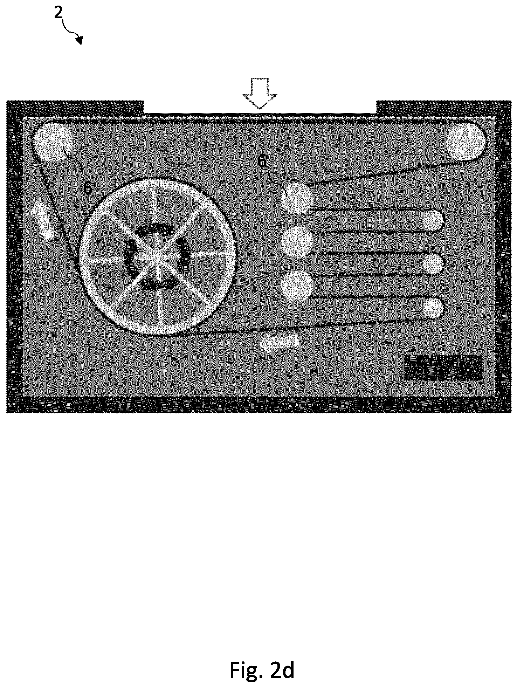

Fig. 2 schematically illustrates a cassette 2 according various embodiments of the disclosure. Fig. 2a is a very similar implementation as that of Fig. 1 apart from composition of the medium that can be handled by the medium handling mechanism 8. As the composition of the medium is different in the implementation shown in Fig. 2a, the medium 18 will be applied to the conveyor 4 differently. The conveyor 4 may be immersed in the medium. In this example, the fluid part of the medium may be only gas or only liquid.

Fig. 2b schematically illustrates a cassette 2 according to yet another embodiment of the disclosure. In this implementation, the driving mechanism 6 is arranged in the medium handling mechanism 8 and the medium handling mechanism 8 is simply a container for the medium 18. Both the driving mechanism 6 and the conveyor 4 are completely immersed in the medium 18. Two optical windows 12 are arranged such that the light beam 14 can be directed from two sides, i.e. the bottom of the conveyor 4 or from the top of the conveyor 4. The two optical windows 12 are arranged such that the conveyor 4 is optically accessible from two sides. This configuration also allows to direct a light beam onto the conveyor (passing through the illumination region 16) from one side while detecting light on the other side of the conveyor. This would allow imaging based on, for example absorption and phase contrast, and it can improve detection efficiency of, for example, scattered light, including Raman signals. This configuration also allows to direct one light beam through the first optical window and another light beam through the second optical window, thus interfering the two light beams in the illumination region.

Fig. 2c schematically illustrates a cassette 2 according yet another embodiment of the disclosure. In this implementation, the driving mechanism comprises only one reel and two pulleys. Thereby, the conveyor may continuously move through the illumination region 16 without changing direction. The speed and direction of motion of the conveyor is controlled by the rotation of the reel.

Fig. 2d schematically illustrates a cassette 2 according to yet another embodiment of the disclosure. In this implementation, the driving mechanism 6 comprises only one reel and a plurality of pulleys. The speed of the conveyor is still controlled by the reel and the length of the conveyor is controlled by the number of pulleys. In order to further extend the length of the conveyor, additional pulleys can be arranged. Longer conveyors allow for larger and faster production of cells as the area for cell growth is increased.

In all implementations, the control unit 10 may be arranged in a separate compartment within the cassette such that it is not immersed in the medium. Alternatively, the control unit may be arranged outside of the main body of the cassette. The control unit typically comprises additional sensors and controllers arranged on the conveyor, on the reels, pulleys, in the medium, etc. These are not illustrated in the figures.

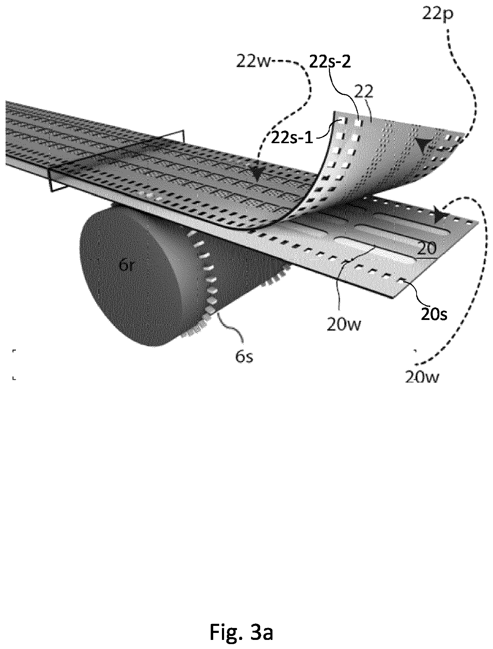

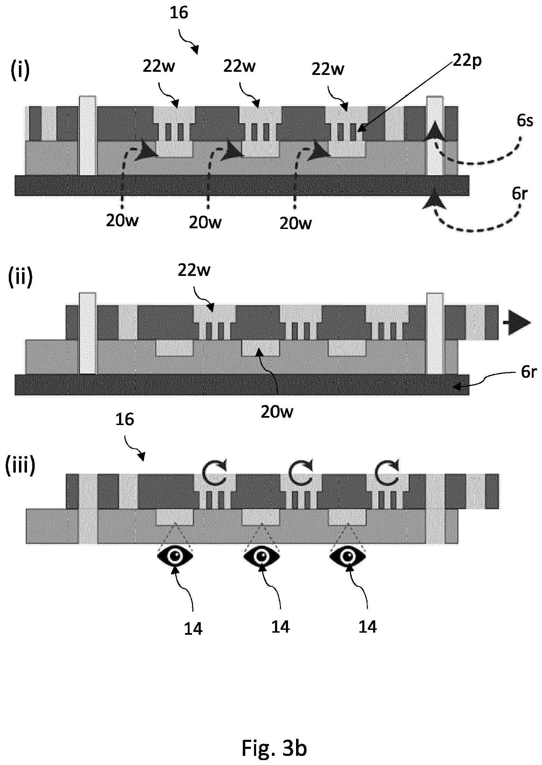

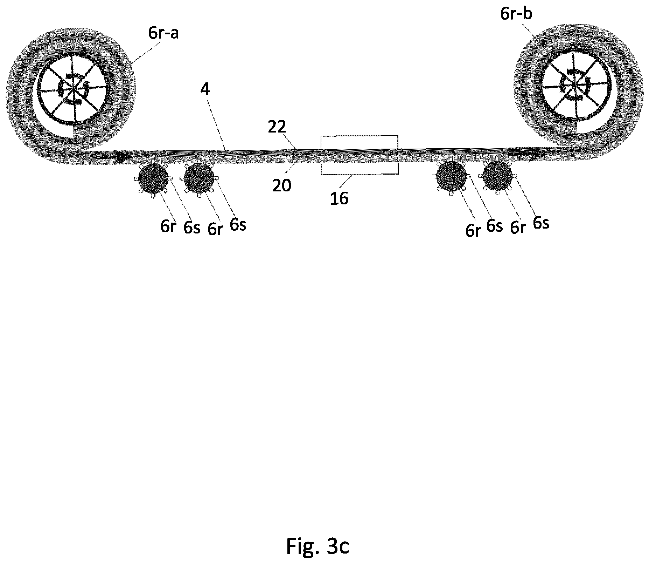

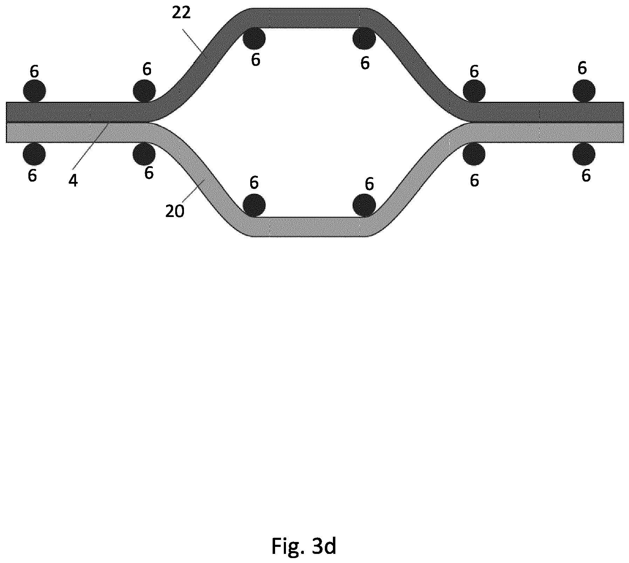

Fig. 3 schematically illustrates a conveyor 4 according to one embodiment of the disclosure. In this embodiment, the conveyor 4 comprises two layers, so-called, a dual layer substrate. In some embodiments, the two layers are configured to capture and guide the medium therebetween. The layers may be configured to move relative to each other, thereby switching between allowing and disallowing medium exchange between layers 20, 22 of the conveyor 4.

Fig. 3a illustrates the conveyor 4 arranged on a roller 6r, the roller comprising sprockets 6s, and being part of the driving mechanism. The conveyor 4 may be made of a flexible and transparent material that is transparent to the imaging light beam and also to any signal elicited by the imaging light beam, e.g., fluorescence. Typically, the conveyor will be transparent in the visible regime 400-700 nm. But, other wavelength ranges may be chosen depending on the light sources and signals being used for imaging. The choice of transmissible wavelength ranges would accommodate the wavelengths of the light sources used for imaging, cell elimination, and reading of position encoders on the conveyor. The layers may be two separate layers. The first layer 20 may be a cell layer. The cell layer 20 may be configured such that the cells may adhere there to. The cell layer 20 may further be configured such that the cells can live and expand in shallow medium-filled wells 20w. The cell layer 20 may be arranged such that it is in contact with rollers and pulleys of the driving mechanism. The second layer 22 may be a cell-free layer, so-called gate layer. The gate layer 22 may be configured to protect cells against sheer stress, while allowing rapid medium exchange. The gate layer 22 may comprise perforations (or pores) 22p configured to enable liquid exchange between the two layers, and in particular to enable liquid exchange in the wells 20w of the cell layer 20. The gate layer 22 may also comprise wells 22w, the gate wells 22w being configured as reservoirs for the medium, which may contain, for example growth factors/reagents/chemicals/signaling molecules, etc. (not shown). Both the cell layer 20 and the gate layer 22 may comprise sprocket holes 20s, 22s, configured to mate with the sprockets on the roller and to thereby enable translation of the conveyor through the cassette. The gate layer 22 may have two rows of sprocket holes 22s-1 and 22s-2 that may allow switching between two configurations, i.e., different spatial alignments of the two layers. The wells 20w of the cell layer may be coated with a material suitable for cell adhesion. The wells 20w may comprise a monolayer and/or clusters of cells comprised in the medium.

Fig. 3b illustrates different conveyor configurations. Fig. 3b(i) illustrates a first configuration when the gate wells 22w are aligned with the cell wells 20w thereby allowing medium exchange between the wells in the two layers via diffusion through the pores 22p in the gate layer 22. In an intermediate stage illustrated in Fig. 3b(ii) there is no medium exchange as the wells of the two layers are not aligned. Fig. 3b(iii) illustrates the conveyor at the illumination region 16 and in an imaging configuration, i.e. when the conveyor has passed the roller 6r. In this configuration the cells are protected from sheer stress, as the conveyor moves rapidly by the driving mechanism. In this configuration the medium may be exchanged within the wells 22w of the gate layer (as indicated by the circular arrow symbol) while the cells are imaged (as indicated by the eye symbol) by the imaging light beam 14 (the eye symbols representing the imaging light beam).

Fig. 3c illustrates how the conveyor 4 may be rolled from one reel 6r-a onto another reel 6r-b while interfacing with sprocket 6s on sprocket rollers 6r along the way. Thus, the two layers (the gate layer 22, and the cell layer 20) of the conveyor 4 may be aligned differently when the conveyor is rolled onto a reel 6r-a and 6r-b, compared to when it is suspended and, for example, traversing the imaging region 16. On the reels 6r-a and 6r-b, the conveyor 4 may be rolled onto itself. Provided an appropriate alignment of wells and pores in the conveyor (not shown) this can allow media exchange also between different segments of the conveyor 4, and not only between different layers 22 and 20.

Fig. 3d illustrates how two layers, i.e. the gate layer 22 and the cell layer 20, of the conveyor 4 may be peeled apart by appropriate positioning of pulleys 6. This enables mechanical interfacing (not shown) with only one part of the conveyor or imaging of only one layer of the conveyor 4.

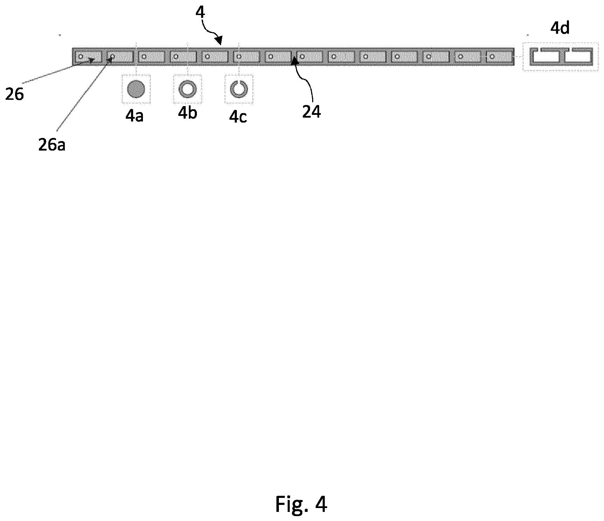

Fig. 4 schematically illustrates a conveyor 4 according to yet one embodiment of the disclosure. The conveyor 4 is in the form of a tube comprising a plurality of walls 24 arranged along the length of the tube-conveyor 4 thereby forming a plurality of pockets 26 along the conveyor 4, the pockets being configured to hold the medium. Each pocket 26 may comprise a perforation 26a configured to allow medium exchange. Inserts 4 a-d illustrate different cross-sections of the tube-conveyor.

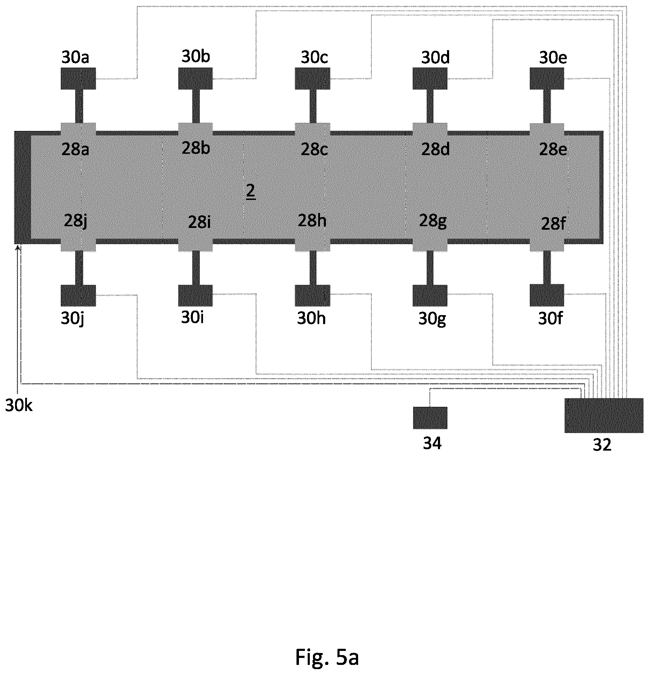

Fig. 5a schematically illustrates a cassette 2 according to yet one embodiment of the disclosure. The cassette 2 comprises a plurality of interface points 28 a-j configured for interfacing the cassette with external systems 30 a-k. The cassette may comprise an interface point 28a which may be configured for cell seeding and thus interfacing the cassette 2 with an auxiliary system for cell seeding 30a. This interface point may be in the form of an inlet for cell seeding. Optionally, the cassette may comprise an interface point 28b configured for cell harvesting and thus interfacing the cassette 2 with an auxiliary system for cell harvesting 30b. The interface point 28b may be in the form of an outlet for cell harvesting. Optionally, the cassette may comprise an interface point 28c configured for temperature control and thus interfacing the cassette 2 with an auxiliary system for temperature control 30c. Optionally, the cassette may comprise an interface point 28d configured for liquid exchange and thus interfacing the cassette 2 with an auxiliary system for liquid handling 30d. The interface point 28d may comprise an inlet for liquid exchange and an outlet for liquid exchange (not shown). Optionally, the cassette may comprise an interface point 28e configured for gas exchange and thus interfacing the cassette 2 with an auxiliary system for gas handling 30e. The interface point 28e may comprise an inlet for gas exchange and an outlet for gas exchange (not shown). Optionally, the cassette may comprise an interface point 28f configured for powering and reading data from sensors in the cassette and thus interfacing the cassette 2 with an auxiliary system for powering sensors and reading sensor-data from sensors in/on the cassette 30f (e.g. temperature sensors). Optionally, the cassette may comprise an interface point 28g configured for reading position encoders on the conveyor and thus interfacing the cassette 2 with an auxiliary system for reading position encoders on the conveyor 30g. The cassette may comprise an interface point 28h configured for additional imaging/screening cells on the conveyor and thus interfacing the cassette 2 with an auxiliary system for imaging/screening cells on the conveyor 30h. The interface point 28h may be an optical access point in the form of one or more glass windows through which light can enter and/or leave the cassette. Optionally, the cassette may comprise an interface point 28i configured for selective removal or elimination of unwanted cells and thus interfacing the cassette 2 with an auxiliary system for selective removal or elimination of unwanted cells 30i. Optionally, the cassette may comprise an interface point 28j configured for inducing motion to the conveyor and thus interfacing the cassette 2 with an auxiliary system for inducing motion to the conveyor 30h. The interface point 28j may be a magnetic interface for inducing motion in the driving mechanism. Optionally, the cassette may be interfaced with an auxiliary system for moving the whole cassette 30k, e.g. for moving the cassette relative to the imaging system. All auxiliary systems 30 a-k may be controlled from a coordination system 32 configured to coordinate all auxiliary systems. The coordinating system 32 may be connected to a system 34 for controlling conditions around the cassette 2.

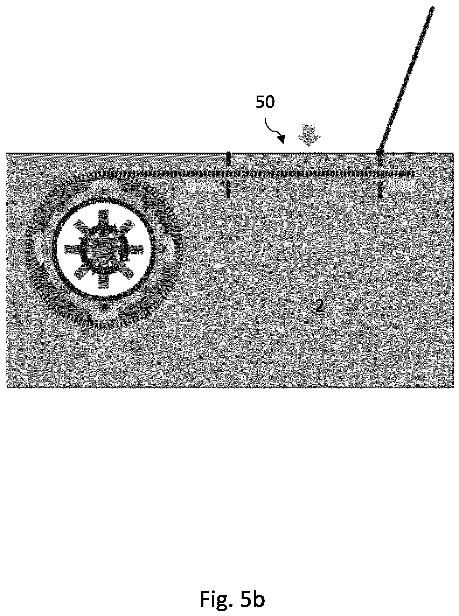

Fig. 5b schematically illustrates a cassette 2 according to yet one embodiment of the disclosure. In this embodiment, the cassette comprises an interface for cell seeding in the form of an open hatch 50 allowing introduction of tools for cell seeding. The cassette 2 may also comprise one or more interface points configured for interfacing the cassette with external systems, similar to those illustrated in Fig. 5a.

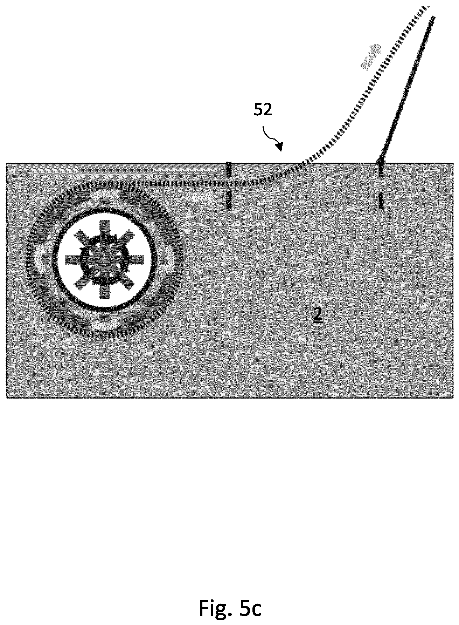

Fig. 5c schematically illustrates a cassette 2 according to yet one embodiment of the disclosure. In this embodiment, the cassette comprises an interface for cell harvesting in the form of an open hatch 52 allowing introduction of tools for cell harvesting and/or extraction of the conveyor. The cassette 2 may also comprise one or more interface points configured for interfacing the cassette with external systems, similar to those illustrated in Fig. 5a.

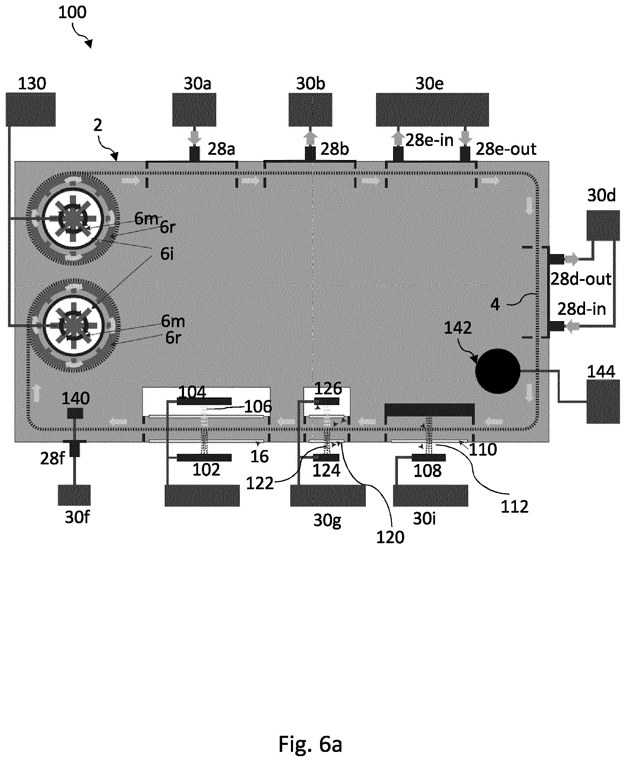

Fig. 6a schematically illustrates a system 100 for production and imaging of bio-cells, the system comprising the cassette 2 according to any embodiment illustrated in previous figures and a first light beam source 102 configured to illuminate at least a portion of the conveyor 4 at a first illumination region 16. The first light beam source 102 is configured to image bio-cells comprised in the cassette 2. The system 100 further comprises a detection means 104 configured to detect light 106 emitted from the first illumination region 16. The system 100 is provided to ensure production as well as qualitative and quantitative analysis of biological cells. The system 100 may further comprise a second light beam source 108 configured to illuminate at least a portion of the conveyor 4 at a second illumination region 110, wherein the second light beam source 108 generates a second light beam 112 configured for removal or elimination of unwanted cells from the cassette 2. The second light source 108 is connected with an auxiliary system for removing or eliminating cells on the conveyor 30i. The unwanted cells may be removed/eliminated by a high-power laser. The system 100 may further comprise further optical means and/or mechanical means and/or magnetic means configured for observing and analyzing the medium in the cassette. The system 100 illustrated in Fig. 6a further includes yet another illumination region 120 for a third light beam 122 generated by the third light source 124 and a detector 126 detecting the light beam emitted from the conveyor. The third light source 124 and the detector 126 are connected with an auxiliary system for reading position encoders on the conveyor 30g thereby enabling reading position encoders on the conveyor 4. The system 100 may further comprise an interface point 28f configured for powering sensors and reading sensor-data from sensors in/on the cassette 2 and thus interfacing the cassette 2 with an auxiliary system for powering sensors and reading sensor-data from sensors in/on the cassette 30f (e.g., temperature sensor 140).

The cassette 2 of the system 100 comprises a driving mechanism 6. The driving mechanism comprises two reels 6r driven by separate rotary motors 6m. The driving mechanism may further comprise additional rollers and/or pulleys, as well as sprocket rollers, which are not illustrated in Fig. 6a. The reels and the motors are interfaced at magnetic interfacing points 6i to thereby induce motion in the driving mechanism. The motors 6m can magnetically interface with the reels 6r or other parts of the driving mechanism 6. The system 100 further comprises a control system 130 for the rotary motors 6m.

The system 100, optionally, may further comprise a cell seeding system 30a connected to a cell seeding inlet 28a provided on the cassette 2. The system may also include a cell harvesting system 30b connected to a cell harvesting outlet 28b provided on the cassette 2. The system 10 may further comprise gas handling system 30e connected to a gas-exchange inlet 28e-in and a gas-exchange outlet 28e-out arranged on the cassette 2. Also, the system 100 may ensure liquid handling by providing the cassette 2 with a liquid exchange inlet 28d-in and a liquid exchange outlet 28d-out connected to an external liquid handling system 30d.

The system 100 may be configured to externally control, e.g., temperature in the cassette by providing the cassette 2 with a thermally conducting interface 142 configured to connect to a temperature control system 144.

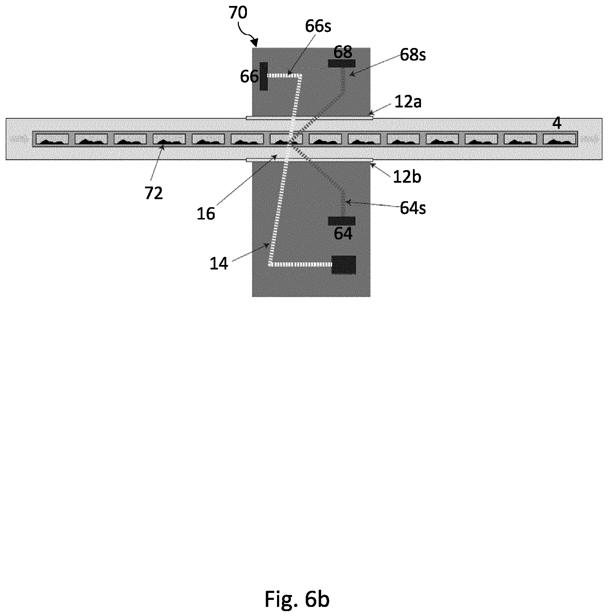

Fig. 6b schematically illustrates an alternative implementation of optical windows and illumination region 16. In this embodiment, there are two optical windows 12a, 12b allowing detection of various signals emitted from the conveyor and the cells. One of the detectors 64 may detect a signal 64s originating from interaction between the imaging light beam 14 and the cells being relayed to the detector 64 in the imaging system 70. Another detector 66 may detect a light beam 66s modulated in, e.g., phase or amplitude or polarization or wavelength upon interaction with the cells and being relayed to the detector 66 in the imaging system through the transparent conveyor 4. The third detector 68 may detect a signal 68s also originating from interaction between the imaging light beam and the cells and which passes through the transparent conveyor 4 and being relayed to the detector 68 in the imaging system. Fig. 6b illustrates only a portion of the conveyor placed inside the cassette (not shown) with cells 72 being present at the bottom of wells of the conveyor 4.

Although particular features have been shown and described, it will be understood that they are not intended to limit the claimed invention, and it will be made obvious to those skilled in the art that various changes and modifications may be made without departing from the scope of the claimed invention. The specification and drawings are, accordingly, to be regarded in an illustrative rather than restrictive sense. The claimed invention is intended to cover all alternatives, modifications and equivalents, as well as combination of various embodiments illustrated in the figures.

### LIST OF REFERENCES

- 2: cassette
- 4: conveyor
- 6: driving mechanism
- 6a: roller
- 8: medium handling mechanism
- 10: control mechanism
- 12: optical window
- 14: imaging light beam
- 16: illumination region
- 18: medium
- 20: cell layer
- 20w: cell layer-well
- 20s: sprocket hole
- 22: gate layer
- 22w: gate layer-well
- 22p: gate layer perforation
- 22s: sprocket hole
- 22s-1: 1^{st} sprocket hole row
- 22s-2: 2^{nd} sprocket hole row
- 24: wall
- 26: pocket
- 28: interface points
- 30: auxiliary systems
- 64, 66, 68: detectors
- 64s, 66s, 68s: signals to be detected
- 70: imaging system
- 72: cells
- 100: system
- 102: first light beam source
- 104: detection means
- 106: emitted light beam
- 108: second light beam source
- 110: second illumination region
- 112: second light beam
- 122: light beam
- 124: third light source
- 126: detector
- 142: thermally conducting interface

## Claims

1. A cassette comprising a conveyor, a driving mechanism, and a medium handling mechanism, wherein
the cassette comprises an optical window configured to allow at least one imaging light beam to illuminate at least a portion of the conveyor at an illumination region,
the medium handling mechanism is configured to provide a medium to the conveyor,
the conveyor being configured to move through the illumination region, wherein the driving mechanism is configured to move the conveyor,
wherein the cassette and the conveyor are configured as an incubator for cells comprised in the medium.

2. The cassette according to claim 1, wherein the conveyor comprises a plurality of layers, wherein at least two layers are configured to capture and guide the medium therebetween.

3. The cassette according to claim 1 or 2, wherein the conveyor comprises wells configured to contain a volume of the medium, wherein the bottom of the wells is configured for attaching the cells comprised in the medium, and wherein the wells are coated with a material suitable for cell adhesion.

4. The cassette according to any of the preceding claims, wherein the conveyor comprises wells and pores, the wells being configured as reservoirs for the medium, the pores being configured to enable a medium exchange within the conveyor.

5. The cassette according to any of the preceding clams 2-4, wherein the layers are configured to move relative to each other, thereby switching between allowing and disallowing medium exchange between layers of the conveyor.

6. The cassette according to any of the preceding claims, wherein the conveyor comprises a plurality of walls arranged along the length of the conveyor thereby forming a plurality of pockets along the conveyor, the pockets being configured to hold the medium.

7. The cassette according to any of the preceding claims, wherein the cassette comprises at least one interface point configured for interfacing the cassette and an external system.

8. The cassette according to any of the preceding claims, wherein the conveyor is configured to bend around the driving mechanism.

9. The cassette according to any of the preceding claims, and wherein the conveyor is a flexible/bendable conveyor belt or a conveyor tube.

10. The cassette according to any of the preceding claims, further comprising a control mechanism, the control mechanism being configured to control environmental parameters in the medium and in the cassette, wherein the control mechanism is configured to control at least one of temperature, pressure, Ph, gas composition, or humidity of gas and/or liquid around the conveyor.

11. The cassette according to any of the preceding claims, wherein the conveyor is configured to contain a monolayer and/or clusters of cells comprised in the medium.

12. The cassette according to any of the preceding claims, wherein the conveyor is configured to allow cells contained in the medium to attach to the conveyor.

13. The cassette according to claims 7-12, wherein the at least one interface point is configured for selective removal of unwanted cells.

14. The cassette according to any of the preceding claims, wherein the cassette is configured to provide an aseptic enclosure for the medium.

15. The cassette according to any of the of the preceding claims, wherein the conveyor is made from a biodegradable material.

16. The cassette according to any of the of the preceding claims, wherein the conveyor comprises embedded point-like features and/or features with predefined shapes, wherein the features are configured to be imaged with an imaging system.
